# EUROPÄISCHE PATENTSCHRIFT

(11) **EP 1 076 551 B1**
(45) Veröffentlichungstag und Bekanntmachung des Hinweises auf die Patenterteilung: **31.07.2002**
(21) Anmeldenummer: 99920851.5
(22) Anmeldetag: 05.05.1999
(51) Int. Cl.: A61K 7/32, A61K 7/48, A61K 7/38, A61K 7/00

(54) **KOSMETISCHE STIFTPRÄPARATE**
COSMETIC PREPARATIONS IN THE FORM OF A STICK
PREPARATIONS COSMETIQUES SOUS FORME DE STICK

(30) Priorität: 14.05.1998 DE 19821691; 18.07.1998 DE 19832425
(43) Veröffentlichungstag der Anmeldung: 21.02.2001
(73) Patentinhaber: Henkel Kommanditgesellschaft auf Aktien, 40589 Düsseldorf (DE)
(72) Erfinder: FÖRSTER, Thomas, D-40699 Erkrath (DE); CLAAS, Marcus, D-40723 Hilden (DE); BANOWSKI, Bernhard, D-40597 Düsseldorf (DE); HEIDE, Barbara, D-47809 Krefeld (DE); WADLE, Armin, D-40699 Erkrath (DE)
(86) Internationale Anmeldenummer: EP9903068
(87) Internationale Veröffentlichungsnummer: WO9959537

(56) Entgegenhaltungen:
- EP-A- 0 295 071
- EP-A- 0 385 763
- DE-A- 19 602 902
- US-A- 4 803 195

## Beschreibung

Die Erfindung betrifft geformte Zubereitungen, bevorzugt in der Form eines Stiftes zum Auftrag wasserlöslicher kosmetischer Wirkstoffe auf die Haut.

Kosmetische und dermatologische Stiftpräparate zur Applikation wasserlöslicher Wirkstoffe kommen üblicherweise in zwei Varianten auf den Markt: Entweder in der Form eines Gels, bei dem der Wirkstoff in einem durch ein Geliermittel, z.B. eine Seife, verfestigten wäßrigen Alkohol oder Polyol gelöst ist, oder in Form von Suspensionsstiften, die in einem wasserfreien, mit Wachs verfestigten Öl, z.B. einem Siliconöl, den Wirkstoff dispergiert enthalten.

Die Seifengel-Stifle haben den Nachteil, daß sie aufgrund des Geliermittels einen alkalischen pH-Wert aufweisen und daher Probleme bei der Einarbeitung saurer Wirkstoffe, z.B. des Aluminiumchlorhydrats oder anderer saurer Antitranspirant-Wirkstoffe bereiten. Die wasserfreien Dispersionsstifte, insbesondere solche auf Basis von flüchtigen Siliconölen haben den Nachteil, daß die dispergierten Wirkstoffe leicht zu sichtbaren Produktrückständen auf der Haut und auf der Kleidung führen. Außerdem sind solche Stifte relativ kostspielig, da die Ölkomponenten als Wirkstoffträger teurer sind als Wasser.

Daneben sind auch emulsionsförmige Stiftzubereitungen beschrieben worden. Aus EP-A-0 291 334 ist z.B. ein transparenter Antitranspirant-Stift bekannt, der hohe Mengen eines flüssigen Öls enthält, das mit Hilfe eines hydrophilen Emulgators und Wasser zu einem transparenten Mikroemulsions-Gel verarbeitet ist. Solche Gele sind aus der Literatur auch als "ringing gel" bekannt und hinterlassen beim Auftrag auf die Haut einen schmierigen Film, der vom Anwender sehr unangenehm empfunden wird. Aus WO 96/06594 war eine Wasser-in-Öl-Antitranspirant-Zusammensetzung bekannt, die jedoch nicht die für ein Stiftpräparat erforderliche Festigkeit und Formbeständigkeit aufweist.

Das Dokument EP-A-295 071 offenbart Antitranspirantstifte mit geringen Reiberückständen, die mehrwertige Alkohole und optional Wasser enthalten, wobei das Mengenverhältnis von mehrwertigem Alkohol zu Wasser größer als 1 ist.

Es bestand daher die Aufgabe, ein kosmetisches Stiftpräparat zu entwickeln, das sich als Träger für wasserlösliche Wirkstoffe oder für Pigmente eignet, das die gewünschte mechanische Festigkeit bei Umgebungstemperatur aufweist und einen ausreichenden, nicht schmierigen Abrieb bei der Applikation auf der Haut hinterläßt, der sich nach Verdunstung des Wassers trocken anfühlt.

In der deutschen Patentanmeldung DE 198 21 691.2 sind bereits Stiftpräparate, die als Dispersionen von Lipid- und Wachskristallen in Wasser vorliegen und durch geringe Mengen hydrophiler Tenside stabilisiert werden, beschrieben. Diese Stiftpräparate sind zwar bezüglich Festigkeit. Abgabevermögen und Kühlwirkung auf der Haut recht befriedigend, es bestand jedoch der Wunsch, das Hautgefühl und die Pflegewirkung noch weiter zu verbessern.

Diese Aufgabe wurde erfindungsgcmäß gelöst durch eine geformte Zubereitung zur Applikation wasserlöslicher Wirkstoffe auf der Haut, bevorzugt in Stift-Form, gekennzeichnet durch einen Gehalt von
25 - 70 Gew.-% einer im Bereich von 25 - 70°C schmelzenden Masse, bestehend aus
   (A) wenigstens 60 Gew.-% eines oder mehrerer polarer Lipide und
   (B) bis zu 40 Gew.-% unpolarer Fettstoffe und ggf. in dieser Masse gelöster kosmetischer oder dermatologischer Wirkstoffe
0,1 - 5 Gew.-% eines wasserlöslichen Tensids
1 - 25 Gew.-% eines wasserlöslichen Wirkstoffs und/oder Lösungsmittels
20 - 60 Gew.-% Wasser oder einer feinteiligen Emulsion oder Mikroemulsion einer Ölkomponente mit einer Tröpfchengröße unter 500 nm.

Die erfindungsgemäßen geformten Zubereitungen sind Dispersionen von Lipid- und Wachskristallen, die durch sehr geringe Mengen von hydrophilen Tensiden stabilisiert werden und die aufgrund ihres Wassergehaltes bei der Anwendung einen frischen, leicht kühlen Eindruck auf der Haut hinterlassen, der sie insbesondere für kosmetische Anwendungen im Bereich der Deodorantien sehr geeignet macht. Die Struktur ist fest und stabil genug, um daraus Stifte zu formen und das Schmelzverhalten ist einerseits durch die Art der Lipide und Fettstoffe, andererseits durch die Struktur und Zusammensetzung der Zubereitungen so beschaffen, daß beim Bestreichen der Haut durch die Körpertemperatur ein Schmelzvorgang eingeleitet wird, der eine gleichmäßige Abgabe der Stiftmasse an die Haut ermöglicht. Darüber hinaus wird durch die feinstemulgierten Ölkomponenten, die bevorzugt in einer Menge von 0.1 - 10 Gew.-% in den Zubereitungen enthalten sind, ein glattes und kosmetisch befriedigendes Hautgefühl und eine gute Pflegewirkung auf der Haut erzielt.

Die erfindungsgemäßen Zubereitungen eignen sich insbesondere, um kosmetische oder dermatologische Wirkstoffe auf die Haut zu bringen. Es kann sich dabei entweder um lipophile. fettlösliche Wirkstoffe handeln, die in der Lipidphase gelöst werden, oder um wasserlösliche Wirkstoffe. Letztere stellen für konventionelle Stiftmassen ein besonderes Problem dar, da diese entweder wasserfrei sind oder mit Seifen verfestigte Gele darstellen, die mit zahlreichen wasserlöslichen Wirkstoffen unverträglich sind. Die erfindungsgemäßen Zubereitungen enthalten aber eine äußere wäßrige Phase, in der beliebige wasserlösliche Wirkstoffe gelöst sein können.

Die dispergierte Lipidphase, die aus wenigstens 60 Gew.-% eines oder mehrerer polarer Lipide (A) und bis zu 40 Gew.-% unpolarer Fettstoffe besteht, sollte bei 25° C fest sein, aber im Bereich von 25 - 70°C schmelzen, wobei es bevorzugt ist, wenn ein Teil dieser Phase bereits bei Temperaturen unter 35°C zu schmelzen beginnt.

Als polare Lipide (A) werden dabei Fettstotte verstanden, die in Wasser weitgehend unlöslich sind, aber polare Gruppen. z.B. Hydroxylgruppen oder Carboxylgruppen sowie lipophile, bevorzugt lineare Alkyl- oder Acylgruppen mit 8 - 22 C-Atomen aufweisen. Beispiele solcher geeigneter polarer Lipide sind z.B. Fettsäuren oder Fettalkohole.

Fettsäuremonoethanolamide oder Fettsäuremonoisopropanolamide, Fettsäure-mono- oder Diglyceride, Fettsäuremono- oder -diester des Sorbitans oder Methylglucosids, jeweils von Fettsäuren mit 8 - 22 C-Atomen.

Bevorzugte polare Lipide zur Herstellung der erfindungsgemäßen Zubereitungen sind ausgewählt aus Fettsäuren oder Fettalkoholen mit 12 - 22 C-Atomen, Partialestern von Fettsäuren mit 12 - 22 C-Atomen und Glycolen oder Polyolen mit 2 - 10 C-Atomen, Fettsäuremonoalkanolamiden mit 12 - 22 C-Atomen im Acylrest und 2 - 4 C-Atomen im Alkanolrest.

Geeignet sind auch die technisch leicht zugänglichen, durch Veresterung von Glycerin oder Sorbit mit 1 - 2 Mol Fettsäure erhältlichen Estergemische. Weitere geeignete polare Lipide sind die Fettsäuremono- und Diestergemische des Methylglucosids oder des Butylglucosids oder des Diglycerins sowie Fettalkohole mit 16 - 40 C-Atomen..

Als unpolare Fettstoffe (B) eignen sich alle bekannten physiologisch verträglichen Fette, Wachse und Paraffine. Geeignete Wachse sind z.B. Bienenwachs, Carnaubawachs, Candclillawachs, Walrat oder synthetische Fettsäure-Fettalkoholester wie z.B. Cetylpalmitat oder Stearylstearat. Bevorzugt geeignete unpolare Fettstoffe sind vor allem Paraffinwachse oder Triglyceride von gesättigten C₁₀-C₂₂-Fettsäuren oder von Hydroxystearinsäure. Beispiele für gesättigte Triglyceride sind vor allem gehärtete Triglyceridfette, z.B. geh. Kokosöl, geh. Palmöl oder geh. Ricinusöl. Andere geeignete unpolare Fettstoffe sind die synthetischen Vollester aus Fettsäuren und Glycolen oder Polyolen mit 2 - 6 C-Atomen z.B. 1,2-Propylenglycol-distearat oder Ethylenglycoldipalmitat sowie Vollester aus Fettalkoholen und Di- oder Tricarbonsäuren, z.B. Dicetylsuccinat oder Dicetyl-/stearyl-adipat.

Es ist nicht erforderlich, daß die Komponenten der bei 25 - 70°C schmelzenden Masse ebenfalls innerhalb dieses Temperaturbereiches schmelzen, vielmehr kann eine Komponente oder ein Teil einer Komponente auch bei 25°C noch flüssig sein. Wichtig ist, daß die Masse der Lipide (A + B) sowie der gegebenenfalls enthaltenen lipophilen Wirkstoffe im Gemisch ein Schmelzverhalten zeigt, das bei 25 - 35°C beginnt und bis 70°C zu einer klaren Schmelze führt.

Als lipophile, öllösliche Wirkstoffe können z.B. öllösliche Vitamine, z.B. Tocopherole, Retinol-Derivate, öllösliche Deodorantien, z.B. Triethylcitrat, öllösliche UV-Filtersubstanzen sowie alle bekannten in der Lipidschmelze löslichen kosmetischen oder dermatologischen Wirkstoffe enthalten sein.

Als wasserlösliche Tenside sind prinzipiell alle oberflächenaktiven Stoffe geeignet, die in Wasser bei 20°C eine Löslichkeit von wenigstens 1 Gew.-%, bevorzugt aber von 10 Gew.-%, aufweisen. Es ist dabei gleichgültig, ob diese Tenside nichtionogen oder ionogen, z.B. anionisch, zwitterionisch, amphoter oder kationisch sind. Gemeinsames Merkmal solcher Tenside ist eine bevorzugt lineare Alkyl-, Alkenyl- oder Acylgruppe mit 8 - 18 C-Atomen sowie eine löslichmachende Gruppe, z.B. eine Sulfat- oder Sulfonat-Gruppe, eine Carboxylat-Gruppe, eine Polyol- oder Polyglycolether-Gruppe oder eine Aminoxid-Gruppe. Geeignete anionische Tenside sind z.B. Alkylsulfate und Alkansulfonate, α-Olefinsulfonate, Acylisethionate, Acyltauride und Acylsarkosinate sowie Alkylpolyglycolethersulfate, alle in Form ihrer Alkali-, Ammonium- oder Alkanolammonium-Salze.

Geeignete zwitterionische Tenside sind z.B. Betaintenside wie Alkyl-dimethylacetobetain oder Acylamidopropyl-dimethylacetobetain.

Geeignete Amphotenside sind z.B. N-Alkylaminopropionsäure oder N-Alkylaminobuttersäure.

Bevorzugt geeignete wasserlösliche Tenside für die Zwecke der erfindungsgemäßen geformten Zubereitungen sind nichtionogene Tenside, z.B. Anlagerungsprodukte von 10 - 30 Mol Ethylenoxid an Fettalkohole, Fettsäuren, Fettsäurealkanolamide, Fettsäuremonoglyceride, Sorbitanfettsäureester, Methylglucosid-Fettsäureester oder polyglycolethermodifizierte Polysiloxane. Besonders bevorzugt sind solche nichtionischen Tenside, die frei von Glycolethergruppen sind und deren hydrophile Gruppe durch einen Oligoglucosidrest gebildet wird. Solche Tenside stehen als Alkylglucoside, Alkyloligoglucoside oder Alkylpolyglucoside unter dem Warenzeichen Plantacare® zur Verfügung. Sie enthalten eine glucosidisch gebundene C₁₀-C₁₆-Alkylgruppe an einem (Oligo)-glucosidrest, dessen mittlerer Oligomerisationsgrad bei 1 - 2 liegt. Auch die vom Glucamin abgeleiteten Acylglucamide sind als Tenside geeignet.

Als wasserlösliche Wirkstoffe können alle kosmetisch oder dermatologisch wirksamen Stoffe eingesetzt werden, die in Wasser bei 20°C zu wenigstens 1 Gew.-% klar löslich sind. Solche Stoffe sind z.B. anionische oder kationische Wirkstoffe, die in Salzform wasserlöslich sind, z.B. Vitamine wie Biotin (Vitamin H), Ascorbinsäure (Vitamin C), Tretinoin (Vitamin-A-Säure), Pantothensäure oder Sonnenschutzwirkstoffe wie 2-Phenylbenzimidazol-5-sulfonsäure, 2-Hydroxy-4-methoxy-benzophenon-5-sulfonsäure, Hautbefeuchtungsmittel wie z.B. DL-2-Pyrrolidon-5-carbonsäure oder Chitosan-Salze, antimikrobielle Stoffe wie z.B. Chlorhexidin-gluconat. Benzoesäure, Sorbinsäure und keratolytische Stoffe wie z.B. Na-Thioglycolat, Sebostatika, Hautaufheller wie z.B. Ascorbylphosphat und Anti-Akne-Wirkstoffe.

Weitere wasserlösliche kosmetische Wirkstoffe sind z.B. anorganische Salze wie z.B. Alaun (K Al(SO₄)₂ 12H₂O), der als blutstillender und deodorierender Stoff eingesetzt wird. Als solche sind bevorzugt keratinhärtende schweißhemmende Salze, insbesondere Aluminiumsalze, enthalten. Außcr dem bereits genannten Alaun eignen sich hierfür z.B. Aluminiumhydroxychlorid, Aluminiumlactat, Natriumaluminium-Chlorhydroxylactat, Aluminiumchlorhydroxyallantoinat, Aluminium-Zirkon-Tetrachlorhydrat-Glycinkomplexe oder Gemische davon.

Weitere wasserlösliche Wirkstoffe, die mit Hilfe der erfindungsgemäßen Zubereitungen auf die Haut gebracht werden können, sind z.B. Hautpigmentierungsmittel und Färbemittel, z.B. Dihydroxyaceton, Alloxan oder 5,6-Dihydroxyindolin-Salze.

Auch entzündungshemmende und die Heilung fördernde Stoffe wie z.B. Allantoin, Panthenol und verschiedene wäßrige oder wasserlösliche Pflanzenextrakte, Proteinhydrolysate und Harnstoff können als wasserlösliche Wirkstoffe eingesetzt werden.

Als Pigmente können z.B. Titandioxid, Kaolin, Talkum, Zinkoxid, Zinkstearat, Calciumstearat, Magnesiumstearat, Magnesiumsilikat, Bariumsulfat und Farbpigmente, z.B. Eisenoxid-Pigmente enthalten sein.

Schließlich kann die erfindungsgemäße Zubereitung neben dem Wasser auch wasserlösliche Lösungsmittel enthalten, die aber in ihrer Menge nicht den Gehalt an Wasser übersteigen sollten. Als wasserlösliche Solventien eignen sich vor allem die niederen Alkohole mit 1 - 3 C-Atomen, die Glycole und Polyole sowie die Polyalkylenglycole, die mit Wasser im Verhältnis 1 : 1 bei 20°C klar mischbar sind, Geeignete Solventien sind z.B. Ethanol, Isopropanol, 1,2-Propylenglycol, Sorbit, Glycerin, Diglycerin oder Polyethylenglycole mit Molgewichten von 200 - 10000.

Durch den Anteil des wasserlöslichen Solvens lassen sich die Festigkeit, das Abgabevermögen (Abrieb an der Haut) und das Hautgefühl sehr gut steuern. So wirken Zubereitungen, die niedere Alkohole wie z.B. Ethanol oder Isopropanol enthalten, oder die einen hohen Wassergehalt aufweisen. angenehm kühlend auf der Haut. Bevorzugt sind in den erfindungsgemäßen geformten Zubereitungen 25 - 60 Gew.-% Wasser oder eines Gemisches aus Wasser und bis zu 10 Gew.-% eines wasserlöslichen Glycols oder Polyols mit 2 - 6 C-Atomen enthalten.

Der Abrieb, d.h. die beim Bestreichen an die Haut abgegebene Menge kann man durch das Mengenverhältnis von polaren und unpolaren Lipiden, dem Gehalt an wasserlöslichem Tensid und dem Verhältnis von Lipidphase zu Wasserphase steuern. Diese Mengenverhältnisse haben auch Einfluß auf das Schmelzverhalten der Stiftmasse, das zwar überwiegend - aber nicht ausschließlich - durch das Schmelzverhalten der Lipidphase bestimmt wird.

Zur Verbesserung des Hautgefühls und der Pflegewirkung kann man den erfindungsgemäßen Zubereitungen auch kosmetische Ölkomponenten zusetzen. Dies kann jedoch nur in begrenztem Umfang durch Zusatz zur Lipidmasse oder durch getrenntes Einemulgieren erfolgen, ohne daß die Stabilität des Systems gefährdet oder gar zerstört wird. Es hat sich aber gezeigt, daß solche Ölkomponenten in Form einer besonders feinteiligen Öl-in-Wasser-Emulsion mit einer Tröpfchengröße unter 500 nm, bevorzugt unter 300 nm, in einer Menge von 0,1 - 10 Gew.-% problemlos in der Wärme und vor dem Erstarren in die Zusammensetzang eingearbeitet werden können.

Als Ölkomponenten eignen sich alle wasserunlöslichen, hautverträglichen Öle und Fettstoffe, die bei Raumtemperatur flüssig sind, zumindest aber einen Schmelzpunkt unterhalb von 40° C aufweisen. Bevorzugt geeignet sind die bei 20° C noch flüssigen Kohlenwasserstoffe, z.B. Paraffinöle oder Polyolefine und synthetische Kohlenwasserstoffe wie z.B. 1,3-Di-(2-ethyl-hexyl)-cyclohexan (Cetiol®S). Eine weiterhin sehr bevorzugte Ölkomponente sind die Di-n-alkylether mit insgesamt 12 - 24 C-Atomen wie z.B. Di-n-octylether, Di-(2-ethylhexyl)-ether, Lauryl-methylether oder Octylbutylether.

Eine besonders vielseitige Gruppe von kosmetischen Ölkomponenten ist die der Fettsäureund Fettalkoholester, z.B. Isopropylmyristat, n-Butylstearat, 2-Ethylhexyl-caprylat, Cetyloleat, Glycerin-tricaprylat, Kokosfettalkohol-(C₁₂-C₁₈)-caprylat-/aprinat, Hexyl-decyl-laurat und andere. Geeignet sind auch natürlich vorkommende Esteröle wie z.B. Jojobaöl oder pflanzliche Triglyceridöle wie z.B. Olivenöl, Sonnenblumenöl. Sojaöl, Rapsöl, Mandelöl sowie die flüssigen Anteile des Kokosöls oder des Rindertalgs sowie synthetische Triglyceridöle. Auch Dicarbonsäureester wie z.B. Di-n-butylsebacat, Di-nbutyl-adipat, Di-isotridecyl-acetat, Di-2-ethylhexyl-succinat eignen sich als Ölkomponente.

Auch lineare und cyclische Polysiloxane (Silikonöle) können als Ölkomponenten enthalten sein. Die Ölkomponenten sind bevorzugt ausgewählt aus flüssigen Kohlenwasserstoffen, Di-n-alkylethern, Fettsäure- oder Fettalkoholestern und Silikonölen.

Schließlich kann die erfindungsgemäße geformte Zubereitung auch noch weitere Hilfs- und Zusatzmittel enthalten, die zur Erzielung der gewünschten Eigenschaften und Wirkungen erforderlich sein können. Es handelt sich dabei aber um übliche Komponenten wie z.B. Duftstoffe, Farbstoffe, Verdickungsmittel. Pigmente, Talkum, Schichtsilikate, Stärken,

Konservierungsmittel und Antioxidantien, die insgesamt in einer Menge von bis zu 10 Gew.-% in der Zubereitung enthalten sein können. So kann z.B. die Stabilität der Zubereitungen während des Herstell- und Abfüllvorganges durch Zugabe wasserquellender Verdickungsmittel (z.B. wasserlösliche Celluloseether, Polyvinylpyrrolidon, Polyvinylalkohol, modifizierter Stärke, hydroxypropylierter Stärkephosphate oder wasserlöslicher Acrylsäure-Acrylat-Copolymere und anderer) merklich verbessert werden. Zur Beeinflussung des Hautgefühls können z.B. Stärken, Talkum, Nanosphären oder Silikonöl in geringen Mengen zugesetzt werden.

Die folgenden Beispiele sollen den Gegenstand der Erfindung näher erläutern:

### Beispiele

### 1. Ölfreie Deodorant-Stifte

Die in der folgenden Tabelle aufgeführten Beispiele für erfindungsgemäße Stiftmassen wurden wie folgt hergestellt:

Polare Lipide und unpolare Fettstoffe (Wachse und Paraffine) und das Tensid wurden zusammengegeben und gemeinsam auf 80°C bis zur klaren Schmelze erwärmt. In diese heiße Fettphase wurde das Gemisch aus Wasser, wasserlöslichen Glycolen und Polyolen und dem Aluminiumhydroxychlorid unter Rühren bei 80°C eingearbeitet. Es bildete sich eine Emulsion, der noch vor dem Erstarren das Parfümöl und ggf. Talkum zugesetzt wurde.

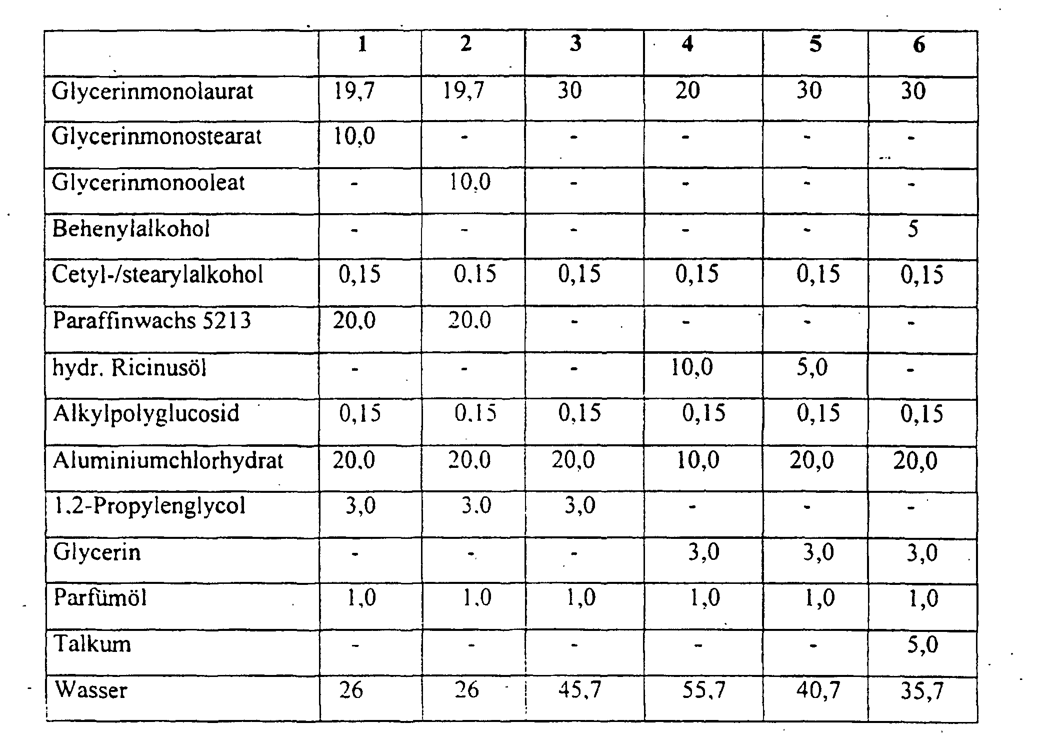

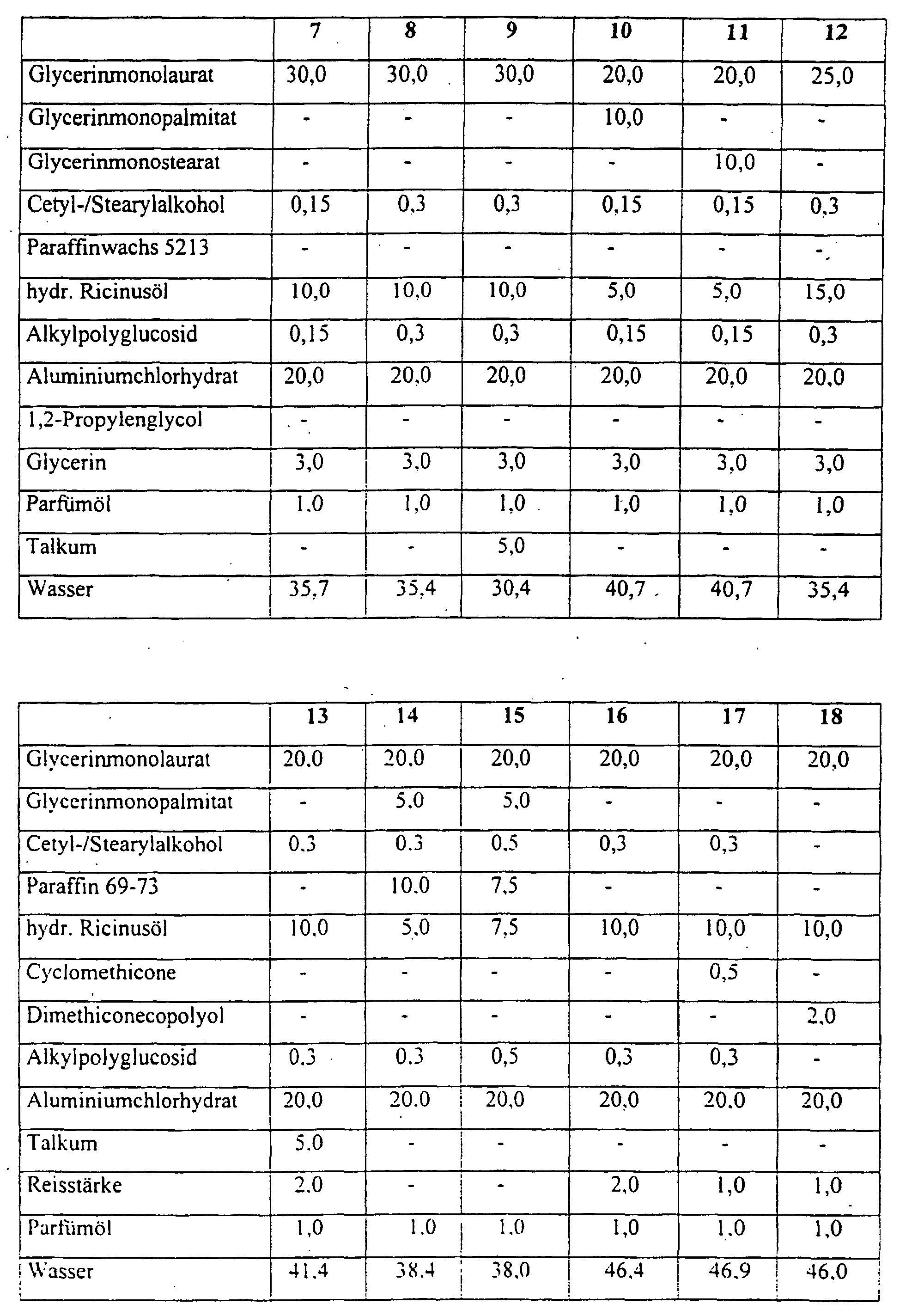

Beim Erreichen der Erstarrungstemperatur bildete sich eine noch fließfähige Dispersion, die ausreichend stabil war, um sie in Stifthülsen abzufüllen. Nach der Abfüllung erstarrten die Massen in der Stifthülse zu einer festen Masse.

In der folgenden Tabelle sind die Schmelzeigenschaften der Beispiele 1 - 18 (nach Messung mit einem Differential Scanning Calorimeter) aufgeführt: Die Unterschiede zwischen den Schmelzmaxima und den Erstarrungsmaxima erklären sich durch Kristallisationsverzögerungen bzw. Neigung zur Unterkühlung.

| **Beispiel** | **Schmelzbeginn [°C]** | **Schmelzmaxima °C** | **Erstarrungsmaxima °C** |
|---|---|---|---|
| 1 | 29 | 37/53 | 30/50 |
| 2 | 26 | 35/54 | 30/5 |
| 3 | 24 | 38 | 11 |
| 4 | 31 | 41/70 | 11/49 |
| 5 | 30 | 41/72 | 13/47 |
| 6 | 29 | 42/73 | 12/33 |
| 7 | 29 | 41/70 | 14/45 |
| 8 | 30 | 42/69 | 14/45 |
| 9 | 31 | 41/67 | 13/57 |
| 10 | 29 | 31/68 | 13/44 |
| 11 | 30 | 42/75 | 14/49 |
| 12 | 30 | 42/75 | 14/49 |
| 13 | 29 | 40/68 | 12/46 |
| 14 | 28 | 38/62 | 14/59 |
| 15 | 28 | 38/62 | 15/58 |
| 16 | 30 | 40/74 | 12/50 |
| 17 | 28 | 40/75 | 12/50 |
| 18 | 29 | 41/68 | 11/52 |

### 2. Herstellung feinteiliger Emulsionskonzentrate für die Einarbeitung von Ölkomponenten in erfindungsgemäße Stift-Zubereitungen

| **2.1 PIT-Emulsion (PIT = 80 - 85°C)** | |
|---|---|
| Hexyl-decyl-laurat | 17.5 |
| Polydecene | 17.5 |
| Cyclomethicone | 10.0 |
| Behenylalkohol-poly-(10)-glycolether | 10.0 |
| Wasser | 45.0 |

| **2.2 Mikroemulsionen** | |
|---|---|
| Di-n-Octylether | 28,0 |
| 2-Octyl-dodecanol | 7,0 |
| Glycerinmonooleat | 5,0 |
| Laurylglucosid | 12,0 |
| Decylglucosid | 8,0 |
| Wasser | 39,0 |

### 3. Antitranspirant-Cremestifte mit Ölkomponenten

| (die %-Angaben der Handelsprodukte beziehen sich auf wasserfreie Aktivsubstanzen) | | | | | | | | | |
|---|---|---|---|---|---|---|---|---|---|
| | **19** | **20** | **21** | **22** | **23** | **24** | **25** | **26** | **27** |
| Monomuls 90-L-12 | 20 | 20 | 20 | 20 | 20 | 20 | 20 | 20 | 20 |
| Cutina HR | 10 | 10 | 10 | - | - | - | - | - | - |
| Lanette 22 | - | - | - | 5 | 7 | 7 | 7 | 7 | 7 |
| Paraffinwachs 69-73 | - | - | - | 8 | 5 | 5 | 5 | 5 | 5 |
| Emulgade PL 68/50 | 1 | 1 | 1 | 2 | 2 | 2 | 2 | 2 | 2 |
| Aluminiumchlorhydrat | 20 | 20 | 10 | 10 | 10 | 10 | 10 | 10 | 10 |
| Talcum Pharma S | - | - | - | 5 | 5 | - | - | - | - |
| Parfümöl | 1 | 1 | 1 | 1 | 1 | 1 | 1 | 1 | 1 |
| PIT-Emulsion 2.1 | 5 | 10 | 3 | 10 | 10 | 10 | 5 | - | - |
| Mikroemulsion 2.2 | - | - | - | - | - | - | - | 3 | 6,4 |
| Wasser | 42 | 38 | 55 | 39 | 40 | 45 | 50 | 52 | 48,6 |

### Herstellung:

Die Herstellung erfolgte analog Beispiel 1 - 18, die Emulsionskonzentrate 2.1 bzw. 2.2 wurden zuletzt während der Abkühlung bei einer Temperatur der Masse von ca. 55°C unter Rühren eingearbeitet.

### Vergleichsversuch:

Es wurde versucht, in die Zusammensetzung des Beispiels 21 anstelle der PIT-Emulsion 2.1 eine grobteilige Emulsion (mittlere Teilchengröße 2000 nm) einzuarbeiten. Dabei wurde eine Masse erhalten, die nicht zu einem festen Stift erstarrte.

### 4. Make-Up Stifte (28 - 31)

### Moisturizer-Stifte (32, 33)

### Hautaufheller-Stifte (34, 35)

| (Die %-Angaben der Handelsprodukte beziehen sich auf wasserfreie Aktivsubstanz) | | | | | | | | |
|---|---|---|---|---|---|---|---|---|
| | **28** | **29** | **30** | **31** | **32** | **33** | **34** | **35** |
| Monomuls 90-L-12 | 20 | 20 | 20 | 20 | 20 | 20 | 20 | 20 |
| Lanette 22 | 8 | 8 | 9 | 9 | 8 | 8 | 8 | 9 |
| Paraffinwachs 69-73 | 5 | 5 | 5 | 5 | 5 | 5 | 5 | 5 |
| Emulgade PL 68/50 | 2 | 2 | 2 | 2 | 2 | 2 | 2 | 2 |
| Talcum Pharma S | 1,5 | 1,5 | 1,5 | 1,5 | - | - | - | - |
| TiO₂ | 3 | 3 | 3 | 3 | - | - | - | - |
| Fe₂O₃ (Braun 70) | 0,7 | 0,7 | 0,7 | 0,7 | - | - | - | - |
| Fe₂O₃ (Braun 75) | 0,25 | 0,25 | 0,25 | 0,25 | - | - | - | - |
| Fe₂O₃(Gelb 10) | 0,10 | 0,10 | 0,10 | 0,10 | - | - | - | - |
| Fe₂O₃ (Rot 30) | 0.02 | 0.02 | 0.02 | 0,02 | - | - | - | - |
| Na-Hyaluronat | - | - | - | - | 0,1 | 0,1 | - | - |
| Herbasol-Extrakt Salbei | - | - | - | - | 1,0 | 2,0 | - | - |
| Aqua Hamamelis | - | - | - | - | 1,0 | 2,0 | - | - |
| Mg-Ascorbylpalmitat | - | - | - | - | - | - | 0,5 | 0,75 |
| PIT-Emulsion 2.1 | 5,0 | 7,5 | 5,0 | 7,5 | 5,0 | 7,5 | 5,0 | 7,5 |
| Konservierungsmittel | 1,55 | 1,55 | 1,55 | 1,55 | 1,55 | 1,55 | 1,55 | 1,55 |
| Parfümöl | 1,0 | 1,0 | 1,0 | 1,0 | 1,0 | 1,0 | 1,0 | 1,0 |
| Wasser | 51,88 | 49,38 | 50,58 | 48,38 | 55,35 | 50,85 | 56,95 | 53,2 |

Die Herstellung erfolgte Analog Beispiel 19 - 27. Die Pigmente wurden nach der PIT-Emulsion 2.1 bei ca. 50°C in die Masse eingearbeitet. Als Konservierungsmittel wurde eine Kombination aus p-Hydroxybenzoesäure-methylester, -propylester und Phenoxyethanol (0,3 + 0,3 + 0,95 Gew.-%) eingesetzt.

Es wurden die folgenden Handelsprodukte verwendet:

| | |
|---|---|
| Abil K4 | Octamethylcyclotetrasiloxan (Cyclomethicone) |
| Abil B 8843 | Dimethiconecopolyol |
| Emulgade PL 68/50 | Gemisch aus Alkyl-(C₁₆₋₁₈)-polyglucosid und Cetyl-/Stearylalkohol (1 : 1) |
| Cetiol PGL | Hexyldecyl-laurat |
| Nexbase 2006 FG | Polydecen |
| Mergital B10 | Behenylalkohol-poly-(10)-glycolether |
| Plantacare 1200 | Laurylglucosid |
| Plantacare 2000 | Decylglucosid |
| Monomuls 90-L-12 | Glycerinmonolaurat |
| Monomuls 90-0-18 | Glycerinmonooleat |
| Cetiol OE | Di-n-octylether |
| Eutanol G | 2-Octyl-dodecanol |
| Cutina HR | Hydr. Ricinusöl |
| Lanette 22 | Behenylalkohol |
| Locron L | Al-chlorhydrat |

## Patentansprüche

1. Geformte Zubereitung zur Applikation wasserlöslicher Wirkstoffe auf der Haut, bevorzugt in Stift-Form, **gekennzeichnet durch** einen Gehalt von
25 - 70 Gew.-% einer im Bereich von 25 - 70°C schmelzenden Masse, bestehend aus
(A) wenigstens 60 Gew.-% eines oder mehrerer polarer Lipide und
(B) bis zu 40 Gew.-% unpolarer Fettstoffe und ggf. in dieser Masse gelöster kosmetischen oder dermatologischen Wirkstoffen
0,1 - 5 Gew.-% eines wasserlöslichen Tensids
1 - 25 Gew.-% eines wasserlöslichen Wirkstoffs und/oder Lösungsmittels
20 - 60 Gew.-% Wasser oder einer feinteiligen Emulsion oder Mikroemulsion einer Ölkomponente mit einer Tröpfchengröße unter 500 nm.

2. Zubereitung gemäß Patentanspruch 1, **dadurch gekennzeichnet, daß** die polaren Lipide (A) ausgewählt sind aus Fettsäuren oder Fettalkoholen mit 12 - 22 C-Atomen, Partialestern von Fettsäuren mit 12 - 22 C-Atomen und Glycolen oder Polyolen mit 2 - 10 C-Atomen. Fettsäuremonoalkanolamiden mit 12 - 22 C-Atomen im Acylrest und 2 - 4 C-Atomen im Alkanolrest.

3. Zubereitungen gemäß Patentanspruch 1 oder 2, **dadurch gekennzeichnet, daß** die polaren Lipide (A) ausgewählt sind aus Fettsäure-(C₁₂-C₁₈)-monoglyceriden und Fettsäure-(C₁₂-C₁₈)-Sorbitanmono- oder -dicstern.

4. Zubereitung gemäß einem der Patentansprüche 1 - 3, **dadurch gekennzeichnet, daß** als unpolare Fettstoffe Paraffinwachs oder Triglyceride von gesättigten C₁₀-C₂₂-Fettsäuren oder von Hydroxystearinsäure enthalten sind.

5. Zubereitung gemäß einem der Patentansprüche 1 - 4, **dadurch gekennzeichnet, daß** als wasserlöslicher Wirkstoff ein keratinhärtendes, schweißhemmendes Salz, bevorzugt ein Aluminiumsalz, enthalten ist.

6. Zubereitung gemäß einem der Patentansprüche 1 - 5, **dadurch gekennzeichnet, daß** 25 bis 60 Gew.-% Wasser oder eines Gemisches aus Wasser und bis zu 10 Gew.-% eines wasserlöslichen Glycols oder Polyols mit 2 - 6 C-Atomen enthalten ist.

7. Zubereitung gemäß einem der Patentansprüche 1 - 6, **dadurch gekennzeichnet, daß** bis zu 10 Gew.-% weiterer Hilfs- und Zusatzmittel enthalten sind.

8. Zubereitung gemäß einem der Patentansprüche 1 - 7, **dadurch gekennzeichnet, daß** sie 0,1 - 10 Gew.-% einer feinteilig emulgierten oder mikroemulgierten Ölkomponente mit einer Tröpfchengröße unter 300 nm enthält.

9. Zubereitung gemäß Anspruch 8, **dadurch gekennzeichnet, daß** die Ölkomponente ausgewählt ist aus flüssigen Kohlenwasserstoffen, Di-n-alkylethern, Fettsäure- oder Fettalkoholestern. Silikonölen oder Gemischen davon.

10. Verfahren zur Herstellung einer Zusammensetzung gemäß Patentanspruch 8 oder 9, **dadurch gekennzeichnet, daß** man die Ölkomponente in Form einer besonders feinteiligen Öl-in-Wasser-Emulsion mit einer Tröpfchengröße unter 300 nm oder in Form einer Mikroemulsion in der Wärme und vor dem Erstarren der Zusammensetzung einarbeitet.

## Claims

1. A shaped preparation, preferably in stick form, for applying water-soluble active principles to the skin, **characterized by** a content of 25 to 70% by weight of a paste melting at 25 to 70°C and consisting of
(A) at least 60% by weight of one or more polar lipids and
(B) up to 40% by weight of nonpolar fatty compounds and optionally cosmetic or dermatological active principles dissolved in the paste,
0.1 to 5% by weight of a water-soluble surfactant,
1 to 25% by weight of a water-soluble active principle and/or solvent,
20 to 60% by weight of water or a fine-droplet emulsion or microemulsion of an oil component with a droplet size below 500 nm.

2. A preparation as claimed in claim 1, **characterized in that** the polar lipids (A) are selected from fatty acids or fatty alcohols containing 12 to 22 carbon atoms, partial esters of fatty acids containing 12 to 22 carbon atoms and glycols or polyols containing 2 to 10 carbon atoms, fatty acid monoalkanolamides containing 12 to 22 carbon atoms in the acyl group and 2 to 4 carbon atoms in the alkanol group.

3. A preparation as claimed in claim 1 or 2, **characterized in that** the polar lipids (A) are selected from fatty acid (C₁₂₋₁₈) monoglycerides and fatty acid (C₁₂₋₁₈) sorbitan monoesters or diesters.

4. A preparation as claimed in any of claims 1 to 3, **characterized in that** paraffin wax or triglycerides of saturated C₁₀₋₂₂ fatty acids or of hydroxystearic acid is/are present as the nonpolar fatty compounds.

5. A preparation as claimed in any of claims 1 to 4, **characterized in that** it contains a keratin-hardening perspiration-inhibiting salt, preferably an aluminium salt, as the water-soluble active principle.

6. A preparation as claimed in any of claims 1 to 5, **characterized in that** it contains 25 to 60% by weight of water or a mixture of water and up to 10% by weight of a water-soluble glycol or polyol containing 2 to 6 carbon atoms.

7. A preparation as claimed in any of claims 1 to 6, **characterized in that** it contains up to 10% by weight of other auxiliaries and additives.

8. A preparation as claimed in any of claims 1 to 7, **characterized in that** it contains 0.1 to 10% by weight of an oil component in the form of a fine-droplet emulsion or microemulsion with a droplet size below 300 nm.

9. A preparations as claimed in claim 8, **characterized in that** the oil component is selected from liquid hydrocarbons, di-n-alkyl ethers, fatty acid or fatty alcohol esters, silicone oils or mixtures thereof.

10. A process for the production of the preparation claimed in claim 8 or 9, **characterized in that** the oil component is incorporated in the form of a particularly fine-droplet oil-in-water emulsion with a droplet size below 300 nm or in the form of a microemulsion at elevated temperature before the preparation solidifies.

## Revendications

1. Préparation moulée pour l'application de principes actifs solubles dans l'eau, sur la peau, de préférence sous forme de stick,
**caractérisée par** une teneur de
- 25-70 % en poids d'une masse fondant dans la zone de 25 à 70°C, formée de,
A) au moins 60 % en poids d'un ou plusieurs lipides polaires, et
B) jusqu'à 40 % en poids de matières grasses non polaires et des principes actifs cosmétiques ou dermatologiques dissous éventuellement dans cette masse.
- 0,1 à 5 % en poids d'un agent tensioactif soluble dans l'eau,
- 1 à 25 % en poids d'un principe actif soluble dans l'eau et/ou d'un solvant,
- 20 à 60 % en poids d'eau ou d'une émulsion finement divisée ou d'une micro-émulsion d'un composant huileux ayant une taille de gouttelettes en dessous de 500 nm.

2. Préparation conformément à la revendication 1,
**caractérisée en ce que**
les lipides polaires (A) sont choisis parmi les acides gras ou les alcools gras ayant de 12 à 22 atomes de carbone, les esters partiels d'acide gras ayant de 12 à 22 atomes de carbone et de glycols ou de polyols ayant de 2 à 10 atomes de carbone, des monoalkanolamides d'acide gras ayant de 12 à 22 atomes de carbone dans le reste acyle et de 2 à 4 atomes de carbone dans le reste alkanol.

3. Préparation conformément à la revendication 1 ou la revendication 2,
**caractérisée en ce que**
les lipides polaires (A) sont choisis parmi les monoglycérides d'acide gras en C₁₂-C₁₈ et les mono- et les di-esters d'acide gras en C₁₂-C₁₈ et de sorbitanne.

4. Préparation conformément à l'une quelconque des revendications 1 à 3,
**caractérisée en ce que**
comme matière grasse non polaire, une cire de paraffine ou des triglycérides d'acides gras saturés en C₁₀-C₂₂ ou de l'acide hydroxystéarique, sont contenus.

5. Préparation conformément à l'une quelconque des revendications 1 à 4,
**caractérisée en ce que**
comme principe actif soluble dans l'eau, un sel qui durcit la kératine, inhibiteur de la transpiration, de préférence un sel d'aluminium est contenu.

6. Préparation conformément à l'une quelconque des revendications 1 à 5,
**caractérisée en ce que**
de 25 à 60 % en poids d'eau ou d'un mélange à base d'eau et de jusqu'à 10 % en poids d'un glycol soluble dans l'eau ou d'un polyol ayant de 2 à 6 atomes de carbone, est contenu.

7. Préparation conformément à l'une quelconque des revendications 1 à 6,
**caractérisée en ce que**
jusqu'à 10 % en poids d'autres adjuvants et additifs sont contenus.

8. Préparation conformément à l'une quelconque des revendications 1 à 7,
**caractérisée en ce qu'**
elle contient de 0,1 à 10 % en poids d'un composant huileux finement émulsionné ou microémulsionné ayant une taille de gouttelettes inférieure à 300 nm.

9. Préparation conformément à la revendication 8,
**caractérisée en ce que**
le composant huileux est choisi parmi les hydrocarbures liquides, les éthers di-n-alkyliques, les esters d'acide gras ou d'alcool gras, les huiles de silicone ou les mélanges de ceux-ci.

10. Procédé de préparation d'une composition conformément à la revendication 8 ou à la revendication 9,
**caractérisé en ce qu'**
on incorpore le composant huileux sous forme d'une émulsion huile dans l'eau particulièrement finement divisée ayant une taille de gouttelette inférieure à 300 nm ou sous forme d'une micro-émulsion, à chaud et avant la solidification de la composition.
